(19) Europäisches Patentamt
European Patent Office
Office européen des brevets

(11) **EP 3 868 444 B1**

(12) **EUROPEAN PATENT SPECIFICATION**

(45) Date of publication and mention
of the grant of the patent:
**15.05.2024 Bulletin 2024/20**

(21) Application number: **21164624.5**

(22) Date of filing: **17.06.2015**

(51) International Patent Classification (IPC):
**A61Q 5/06** (2006.01)  **A61K 8/34** (2006.01)
**A61K 8/41** (2006.01)  **A61K 8/36** (2006.01)
**A61K 8/368** (2006.01)  **A61K 8/37** (2006.01)
**A61K 8/60** (2006.01)  **A61K 8/42** (2006.01)

(52) Cooperative Patent Classification (CPC):
**A61K 8/42; A61K 8/342; A61K 8/361; A61K 8/368;**
**A61K 8/37; A61K 8/375; A61K 8/415; A61K 8/60;**
**A61Q 5/06**

(54) **COMPOSITION FOR HAIR FRIZZ REDUCTION**

ZUSAMMENSETZUNG ZUR HAARKRÄUSELUNGSREDUZIERUNG

COMPOSITION PERMETTANT DE RÉDUIRE LES FRISOTTIS

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **17.06.2014 US 201462013168 P**

(43) Date of publication of application:
**25.08.2021 Bulletin 2021/34**

(62) Document number(s) of the earlier application(s) in
accordance with Art. 76 EPC:
**15735778.1 / 3 157 637**

(73) Proprietor: **The Procter & Gamble Company**
**Cincinnati, OH 45202 (US)**

(72) Inventors:
• **MARSH, Jennifer Mary**
**Cincinnati, OH 45202 (US)**
• **PUNYANI, Supriya**
**138547 Singapore (SG)**
• **JONES, Stevan David**
**Cincinnati, Ohio 45202 (US)**
• **VATTER, Michael Lee**
**Cincinnati, Ohio 45202 (US)**
• **YAGNIK, Chetan Kantilal**
**138648 Singapore (SG)**

(74) Representative: **P&G Patent Germany**
**Procter & Gamble Service GmbH**
**Sulzbacher Straße 40**
**65824 Schwalbach am Taunus (DE)**

(56) References cited:
**JP-A- S63 156 711      JP-A- 2014 097 931**
**US-A1- 2008 038 206    US-A1- 2012 093 751**

Remarks:
The file contains technical information submitted after the application was filed and not included in this specification

**Description**

FIELD OF THE INVENTION

A leave-on composition is provided and comprises one or more materials useful for treating hair frizz. The moisture control material comprises 2-Hexyl-1-decanol in combination with Salicylic acid. The leave-on composition further comprises a gel matrix as defined hereinafter.

BACKGROUND OF THE INVENTION

Hair frizz is described by consumers as the appearance of unruly fibers at the top of the scalp and tips of hair as well as an increased volume through the bulk of the hair. Generally they see this frizz on days when there is humid weather and the level of moisture in the air is high. The appearance of frizz is undesired and it is often associated with a loss of shine and smoothness. The appearance of frizz and loss of shine and smoothness are associated with a perception of poor hair health. The basic mechanism causing frizz in high humid environments is that at high humidity water penetrates into hair and changes the chemical bond interactions inside the hair. During styling, the consumer will create a 'wet set' where hair is blow dried or flat ironed to create the desired shape. During drying, water is evaporated from hair and hydrogen bonds are formed between the protein peptide chains holding the style in place. As moisture diffuses into hair the hydrogen bonds are broken and hair returns back to its natural shape. For consumers who straighten their hair by blow drying or flat ironing this return to a curled style is associated with a loss of alignment and increased volume. In addition, at high moisture levels in hair the fiber diameter increases which also increases the overall volume of hair.

US 2012/0093751 A1 is related to means for improving tactile sensation of hair during application of a hair cosmetic composition, particularly a hair conditioner or a hair rinse, after washing of the hair.

JP 2014-97931 A refers to a hair treatment agent which has high viscosity while reducing the amount of higher alcohol contained therein.

JP S63-156711 A sets out a hair cosmetic capable to imparting high gloss to the hair without giving dry and loose hair, by using a specific quaternary ammonium salt, a specific phosphoric acid ester and a specific branched oil component as essential components as essential components.

US 2008/0038206 A1 is about a product release system for atomizing compositions which has (a) a pressure-resistant packaging, (b) a capillary-containing spray head, and (c) a propellant-containing composition.

The typical strategy to prevent frizz is to formulate leave-on products with surface-depositing materials such as silicone, oils, conditioning silicone etc. which make hair more hydrophobic and decrease inter-fiber interactions. At high levels these materials can also provide increased cohesive forces holding fibers together to prevent frizz from occurring. With these materials depositing on the hair surface a greasy look and feel is typically experienced, which is an undesired trade-off of frizz reduction.

Consequently, a need exists for a treatment product that combines effective frizz control with additional hair benefits that the consumer can notice and feel and, at the same time, is delightful to use without having a sticky or greasy feel.

SUMMARY OF THE INVENTION

An aqueous hair leave-on composition for hair frizz reduction is provided and comprises: from 0.15% to 12.0% of a moisture control material or mixture of moisture control materials wherein the moisture control material comprises 2-Hexyl-1-decanol in combination with Salicylic acid; wherein the leave-on composition further comprises a gel matrix comprising: (i) from 0.1% to 20% of one or more high melting point fatty compounds, by weight of said aqueous hair leave-on composition, wherein the one or more high melting point fatty compounds have a melting point of 25°C or higher; (ii) from 0.1% to 10% a cationic surfactant system, by weight of said aqueous hair leave-on composition; and (iii) at least 20% of an aqueous carrier, by weight of said aqueous hair leave-on composition.

Without being bound by theory, the materials in the leave-on treatment composition as set out herein provide excellent frizz performance without a negatively affecting hair feel. These materials prevent water uptake into hair under high humidity conditions, reducing the negative impact of frizz. By providing frizz benefits by penetrating the hair fiber as opposed to depositing on the hair surface, the frizz benefit is not associated by negative hair feel, which is typically observed with current commercial anti-frizz products. These and additional features provided by the aspects of the aqueous hair leave-on composition will be more fully understood in view of the following detailed description.

**EP 3 868 444 B1**

## DETAILED DESCRIPTION OF THE INVENTION

All percentages and ratios used herein are by weight of the total composition, unless otherwise designated. All measurements are understood to be made at ambient conditions, where "ambient conditions" means conditions at 25 °C, under one atmosphere of pressure, and at 50% relative humidity (RH), unless otherwise designated. All numeric ranges are inclusive of narrower ranges; delineated upper and lower range limits are combinable to create further ranges not explicitly delineated.

The compositions can comprise, consist essentially of, or consist of, the essential components as well as optional ingredients described herein. As used herein, "consisting essentially of" means that the composition or component may include additional ingredients, but only if the additional ingredients do not materially alter the basic and novel characteristics of the claimed compositions or methods.

"Apply" or "application" as used in reference to a composition, means to apply or spread the compositions onto keratinous tissue such as the hair.

"Dermatologically acceptable" means that the compositions or components described are suitable for use in contact with human skin tissue without undue toxicity, incompatibility, instability, allergic response, and the like.

"Safe and effective amount" means an amount of a compound or composition sufficient to significantly induce a positive benefit.

"Leave-on," in reference to compositions, means compositions intended to be applied to and allowed to remain on the keratinous tissue. These leave-on compositions are to be distinguished from compositions, which are applied to the hair and subsequently (in a few minutes or less) removed either by washing, rinsing, wiping, or the like. Leave-on compositions exclude rinse-off applications such as shampoos, rinse-off conditioners, facial cleansers, hand cleansers, body wash, or body cleansers. The leave-on compositions may be substantially free of cleansing or detersive surfactants. For example, "leave-on compositions" may be left on the keratinous tissue for at least 15 minutes. For example, leave-on compositions may comprise less than 1% detersive surfactants, less than 0.5% detersive surfactants, or 0% detersive surfactants. The compositions may, however, contain emulsifying, dispersing or other processing surfactants that are not intended to provide any significant cleansing benefits when applied topically to the hair.

"Soluble" means at least 0.1 g of solute dissolves in 100 ml of solvent, at 25 °C and 1 atm of pressure.

All percentages are by weight of the total composition, unless stated otherwise. All ratios are weight ratios, unless specifically stated otherwise. All ranges are inclusive and combinable. The number of significant digits conveys neither a limitation on the indicated amounts nor on the accuracy of the measurements. The term "molecular weight" or "M.Wt." as used herein refers to the weight average molecular weight unless otherwise stated. The weight average molecular weight may be measured by gel permeation chromatography. "QS" means sufficient quantity for 100%.

The term "substantially free from" or "substantially free of" as used herein means less than 1%, or less than 0.8%, or less than 0.5%, or less than 0.3%, or 0%, by total weight of the composition.

"Hair," as used herein, means mammalian hair including scalp hair, facial hair and body hair, particularly on hair on the human head and scalp.

"Cosmetically acceptable," as used herein, means that the compositions, formulations or components described are suitable for use in contact with human keratinous tissue without undue toxicity, incompatibility, instability, allergic response, and the like. All compositions described herein which have the purpose of being directly applied to keratinous tissue are limited to those being cosmetically acceptable.

"Derivatives," as used herein, includes but is not limited to, amide, ether, ester, amino, carboxyl, acetyl, acid, salt and/or alcohol derivatives of a given compound.

"Polymer," as used herein, means a chemical formed from the polymerisation of two or more monomers. The term "polymer" as used herein shall include all materials made by the polymerisation of monomers as well as natural polymers. Polymers made from only one type of monomer are called homopolymers. Polymers made from two or more different types of monomers are called copolymers. The distribution of the different monomers can be calculated statistically or block-wise - both possibilities are suitable for the aspects disclosed herein. Except if stated otherwise, the term "polymer" used herein includes any type of polymer including homopolymers and copolymers.

The mechanism of action for frizz generation involves moisture from the environment being absorbed by hair and occupying hydrogen bonding sites within hair, including those on the peptide backbone and also associated with acidic and basic side chains of amino acid residues such as lysine, arginine and glutamic acid. This internal water replaces hydrogen bonds that had been created during styling that hold hair in a desired configuration. As a consequence, hair returns to its natural shape which typically leads to unwanted wave, loss of alignment and frizz. In addition, uptake of water by these hydrogen bonding sites swells the hair fiber causing style expansion, which is another indicator of frizz. Without being bound by theory, the moisture control materials as recited herein will replace water at the hydrogen bond sites inside hair and prevent water uptake. Reduction of water inside hair will lead to a reduction in the appearance of frizz under high humidity conditions. Because the mechanism of action is related to the space inside the hair fibers, there are no feel negatives, such as, for example, greasy or oily feel associated with the benefit. The reduction in water

3

uptake is measured using Dynamic Vapor Sorption (DVS) method, which measures a weight increase of hair equilibrated at 0% Relative Humidity (RH) versus 90% RH. Significant frizz benefit is measured on hair treated by materials that caused a reduction in water uptake of higher than 5 % versus control hair that is not treated with such materials. The treatment involved the application of a 2% w/w solution of the material in 50:50 water: ethanol solvent.

Preferred materials include salicylic acid and 2-hexyl-1-decanol. These materials are chosen from Molecular Class I and/or Molecular Class II or can also be used in combination to increase the size of the benefit.

The concentration of the Moisture Control Material or the concentration of the mixture of Moisture Control Material in a hair leave-on composition is from 0.15% to 12%, or from 0.2% to 5%, or from 0.5% to 4%, or from 1.0% to 3.0%.

**Molecular Class I: Polar, acidic compounds with the following properties:** Protein Binding (PB)>20 AND Molecular Volume (Mol. Vol). <500 AND logP < 3 AND Hydrogen-binding (H-binding) > 10 AND pKa < 5.0, wherein PB is % protein binding, Mol. Vol is molecular volume (in $Å^3$); log P is n-octanol/water partition coefficients. These properties can be calculated using Volsurf software (http://www.moldiscoverv.com/soft volsurf.php). H-bond is the energy from hydrogen bonds between molecules from Hansen Solubility Parameters and pKa value is a logarithmic measure of the acid dissociation constant.

| Name (1% wt /vol) | PB | Mol. Vol. | log P | pKa | H-bond (MPa^1/2) | % Water Reduction |
|---|---|---|---|---|---|---|
| 2,4-Dihydroxybenzoic acid | 28 | 324 | 1.5 | 3.2 | 23 | 30 |
| 3-Hydroxybenzoic Acid | 38 | 314 | 1.6 | 4.3 | 20 | 20 |
| Gallic acid | 23 | 337 | 0.9 | 4.4 | 23 | 15 |
| 3-Aminobenzoic acid | 41 | 326 | 0.9 | 3.6 | 16 | 12 |
| 4-Aminobenzoic acid | 42 | 323 | 0.9 | 3.5 | 16 | 12 |
| 2,5-Dihydroxybenzoic acid | 31 | 329 | 1.6 | 2.9 | 23 | 27 |
| 3,4-Dihydroxybenzoic acid | 27 | 327 | 0.9 | 4.4 | 23 | 20 |
| 3,5-Dihydroxybenzoic acid | 27 | 327 | 0.9 | 4.1 | 23 | 15 |
| 2,6-Dihydroxybenzoic acid | 37 | 326 | 1.6 | 2.1 | 23 | 35 |
| 5-Chlorosalicylic acid | 56 | 361 | 2.3 | 3.0 | 21 | 28 |
| Salicylic acid | 44 | 320 | 2.1 | 3.1 | 20 | 18 |
| Trans-Ferulic Acid | 50 | 451 | 1.5 | 4.5 | 19 | 6 |
| p-Coumaric acid | 46 | 391 | 1.6 | 4.5 | 20 | 8.8 |
| 4-Hydroxybenzenesulphonic acid | 55 | 271 | 1.5 | 2.7 | 22 | 26 |
| 3-Chloro-4-hydroxybenzoic acid | 49 | 356 | 2.1 | 4.1 | 20 | 11 |
| 3,5-Dichloro-4-hydroxybenzoic acid | 51 | 397 | 2.8 | 3.8 | 20 | 15 |
| 2,5 Dihydroxyterephthalic acid | 20 | 375 | 1.1 | 2.1 | 22 | 18 |
| 3-Aminophenol | 45 | 284 | 0.6 | 4 | 17 | 14 |
| 3-Hydroxyanilinium chloride | 32 | 280 | 0.6 | 4 | 17 | 16 |
| 2-Aminophenol | 49 | 288 | 1.0 | 4 | 17 | 14 |
| 4-Aminophenol | 39 | 284 | 0.6 | 4 | 17 | 10 |
| N-4-Hydroxyphenylglycine | 37 | 388 | 1.3 | 3 | 13 | 15 |

**b) Molecular Class II:** Weakly polar to non-polar, weakly to non-acidic compounds that have the following properties: PB>10 AND Mol. Vol. < 1500 AND log P > 0.5 AND pKa ≥ 5 AND H-binding > 4, wherein PB is % protein binding, Mol. Vol is molecular volume (in $Å^3$); logP is n-octanol/water partition coefficients. These properties can be calculated using Volsurf software (http://www.moldiscovery.com/soft_volsurf.php). H-bond is the energy from hydrogen bonds between molecules from Hansen Solubility Parameters and pKa value is a logarithmic measure of the acid dissociation constant.

| Name | PB | Mol. Vol. | logP | pKa | H-bond (MPa^1/2) | % water reduction |
|---|---|---|---|---|---|---|
| 2-Hy droxy ethyl salicylate | 45 | 419 | 1.5 | 8.3 | 19.1 | 10 |
| Ethyl gallate | 43 | 431 | 1.4 | 8.7 | 22.6 | 17 |
| Oleic Acid | 100 | 832 | 7 | 5 | 6.4 | 14 |
| Ricinoleic acid | 84 | 841 | 5.9 | 5 | 17.8 | 8.8 |
| Isovaleric acid | 29 | 295 | 1.3 | 5 | 9.7 | 15 |
| Isobutyric acid | 15 | 254 | 1 | 5 | 10.4 | 5 |
| 2-Hexyl-1-decanol | 87 | 745 | 6.8 | 15 | 10.1 | 11 |
| Phytol | 100 | 874 | 8.0 | 13 | 9.6 | 14 |
| Sorbitan caprylate | 32 | 695 | 1.3 | 12 | 21.8 | 11 |
| Glyceryl monooleate | 96 | 974 | 6.27 | 12.8 | 16.2 | 5 |
| Isostearyl isostearate | 100 | 1527 | 14.7 | 14 | 4.2 | 11 |
| Ethyl linoleate | 82 | 903 | 7.71 | 7.8 | 5.1 | 8 |
| Isopropyl myristate | 97 | 798 | 6.99 | 8.8 | 5.0 | 12 |
| Octyl salicylate | 82 | 646 | 5.4 | 7.1 | 11.7 | 14 |

pH of Compositions

The table below demonstrates data of the difference of % water reduction of hair treated with leave on composition containing 1% salicylic acid in ethanol: water (50:50) at various values of pH vs control (hair treated with composition of ethanol: water (50:50) . As shown in below table, at lower pH, improved performance of the recited composition is obtained compared to higher pH.

| Formula Example not within the scope of the present invention | pH 3 | pH 4.2 | pH 7 | pH 10 |
|---|---|---|---|---|
| **Raw Material** | | | | |
| Distilled Water | QS | QS | QS | QS |
| Ethanol | 50.0 | 50.0 | 50.0 | 50.0 |
| Salicylic acid | 1.0 | 1.0 | 1.0 | 1.0 |
| Final pH | 3.2 | 4.2 | 7 | 10 |
| % Water Reduction | 30 | 27 | 22 | 15 |

The pH of a composition comprising material from Molecular Class I may be in the range of from 1 to 9, or a pH of from 2 to 7, or a pH of from 4 to 5.5.

The pH of a composition comprising materials from Molecular Class II may be in the range of from 1 to 9, or a pH of from 2 to 8, or a pH of from 3 to 7.

The Moisture control Material is a carboxylic acid ester. The carboxylic acid ester is based on a fatty acid wherein the molecule of the fatty acid comprises of more than 14 carbon atoms. Non-limiting examples of such esters are isostearyl isostearate, methyl stearate, methyl palmitate, and methyl oleate. The carboxylic acid ester is part of a mixture of materials prepared via the reaction of natural oils using methanol. Non-limiting examples of such mixture is the mixture that is produced by the product of the reaction of refined palm kernel oil with methanol, followed by fractionation via distillation. A commercial product that meets this description is the Heavy Cut Ester CE-1875 (supplied by P&G Chemicals with CAS Number 6772-38-3) containing ingredients such as methyl stearate, methyl palmitate, methyl oleate as major ingredients, as well as methyl laurate, methyl myristate, methyl behenate and other materials as minor ingredients.

FORMULATIONS AND EXAMPLES

The following examples are non-limiting examples of the recited aqueous hair leave-on composition. The examples are given solely for the purpose of illustration and are not to be construed as limitations of the aqueous hair leave-on compositions.

Examples

***Leave-on Treatment Composition Preparation:***

The leave-on treatment compositions are prepared by adding the Moisture Control Materials and perfume, if needed, into a 50:50 ethanol/water carrier and stirred until complete dissolution. The solution pH is adjusted using sodium hydroxide (50% w/w) to a final pH of 4.0-4.2. The Sepigel 305 is then added, if needed, and the solution is mixed using a high-speed-mixer for 2-5 minutes at 1800-2300 rpm until a uniform composition is obtained.

***Leave- on Hair Treatment Protocol:***

An amount of 0.20 g of each composition of Examples I to IV is spread via a syringe onto separate natural virgin brown hair switches weighing 2.0 g (dosage 0.10g of solution per g of hair). The hair is allowed to air dry and then analyzed using the DVS method described above. The experiment is repeated for a dosage of 0.50 g of solution per g of hair. The hair in this case is also assessed by expert graders, as described below, in addition to the DVS analysis.

***DVS Measurement:***

An amount of 25-30 mg of hair with length of approximately 1 cm is weighed and hold for equilibration at 0% RH for 16 hours. After the 16-hour period, the RH is increased to 10% and maintained at this level for 6 hours. Then, the RH is increased by 10% after every 6 hours interval until it reaches 90% RH. The % water reduction is calculated as follows:

A = Amount of water absorbed by the hair treated with composition containing the Moisture Control Material
B = Amount of water absorbed by the hair treated with control composition (only carrier) containing no Moisture Control Material

$$\text{\% Water reduction} = [(B\text{-}A) \times 100] / B$$

**Hair Switch Feel Assessment Method:** The treated hair switches are kept at high humidity (above 85% RH) for 2 hrs and then ten expert graders are asked to rate each of them in terms of tactile feel based on a 5 point scale, 5 being the highest (best feel) and 1 being the lowest rating.

***Leave-on Treatment Formulation***: Examples I-IV and Examples VI-VII below are not within the scope of the invention.

| Formula Example | Leave-on treatment Control | I | II | III | IV | V | VI | VII |
|---|---|---|---|---|---|---|---|---|
| Raw Material | (wt. / wt.)% | (wt. / wt.)% | (wt. / wt.)% | (wt. / wt.)% | (wt. / wt.)% | (wt. / wt.)% | (wt. / wt.)% | (wt. / wt.)% |
| Distilled Water | QS | QS | QS | QS | QS | QS | QS | QS |
| Ethanol | 50.00 | 50.00 | 50.00 | 50.00 | 50.00 | 50.00 | 50.00 | 50.00 |
| Polyacrylamide & C13-14 Isoparaffin & Laureth-7 (Sepigel 305) | 1.85 | 1.85 | 1.85 | 1.85 | 1.85 | 1.85 | 1.85 | 1.85 |
| Perfume | 0.46 | 0.46 | 0.46 | 0.46 | 0.46 | 0.46 | 0.46 | 0.46 |
| Salicylic acid | 0 | 2.0 | 0 | 0 | 2.0 | 2.0 | 0.0 | 0.0 |

(continued)

| Formula Example | Leave-on treatment Control | I | II | III | IV | V | VI | VII |
|---|---|---|---|---|---|---|---|---|
| Raw Material | (wt. / wt.)% | (wt. / wt.)% | (wt. / wt.)% | (wt. / wt.)% | (wt. / wt.)% | (wt. / wt.)% | (wt. / wt.)% | (wt. / wt.)% |
| 5-Chlorosalicylic acid | 0 | 0 | 2.0 | 0 | 0 | 0 | 2.0 | 2.0 |
| 2, 4-Dihydroxybenzoic acid | 0 | 0 | 0 | 2.0 | 0.15 | 0.15 | 0.15 | 0.15 |
| Oleic acid | 0 | 0 | 0 | 0 | 0 | 0.25 | 0 | 0.25 |
| 2-Hexyl-1-decanol | 0 | 0 | 0 | 0 | 0 | 0.25 | 0 | 0.25 |
| Final pH | 4.2 | 4.2 | 4.2 | 4.2 | 4.2 | 4.2 | 4.2 | 4.2 |
| % Water Reduction versus Leave-on Treatment Control at dose of 0.10g of composition for 1.0g of hair | '- | - | - | - | 4 | 5 | 5 | 7 |
| % Water Reduction versus Leave-on Treatment Control at dose of 0.50g of composition for 1.0 g of hair. Control is dosed at 0.50g of composition for 1.0g of hair | - | 4 | 5 | 5 | 9 | 8 | 10 | 10 |
| Feel Rating Leave-on Treatment Control at dose of 0.10g of composition for 1.0g of hair | 1 | 1 | 2 | 2 | 2 | 3 | 2 | 4 |

Examples VIII-XIII below are not within the scope of the invention.

| Formula Example | VIII | IX | X | XI | XII | XIII |
|---|---|---|---|---|---|---|
| Raw Material | | | | | | |
| Distilled Water | QS | QS | QS | QS | QS | QS |
| Ethanol | 50.0 | 50.0 | 50.0 | 50.0 | 50.0 | 50.0 |
| 5-Chlorosalicylic acid | 1.0 | | | 1.0 | 1.0 | 1.0 |
| 2-Hexyl-1-decanol | | | 5.0 | 5.0 | | 5.0 |
| Isostearyl isostearate | | 2.0 | | | 2.0 | 2.0 |
| Final pH | 4 | 4 | 4 | 4 | 4 | 4 |
| % Water Reduction versus Leave-on Treatment Control at dose of 0.10g of composition for 1.0g of hair | 1.3 | 0.7 | 1.0 | 2.0 | 1.4 | 3.0 |
| Feel Rating (on 5 scale point with 5 as highest and 1 as lowest) | 1 | 2 | 2 | 3 | 3 | 4 |

**Results:** Comparative Formula I to XIII showed % water reduction at high humidity. Higher % water reductions are observed in hair treated with higher doses of leave-on Formulas I-XIII.

The feel assessment results indicate that combinations of

(a) 5-Chlorosalicylic acid and 2-hexyl-1-decanol;
(b) 5-Chlorosalicylic acid and isostearyl isostearate;
(c) 5-Chlorosalicylic acid and 2-hexyl-1-decanol and isostearyl isostearate

provide, not only water absorption reduction (resulting in frizz benefit), but also tactile feel benefit. This is shown by the feel comparisons of (a) Example XI versus Examples VIII and IX, (b) Example XII versus Examples VIII and X, and (c) Example XIII versus Examples VIII, IX and X.

Additional Evaluations not within the scope of the invention

Additional leave-on treatment compositions are prepared (Tables 1 and 2) according to the procedure described above, which are used to treat hair switches using the procedure described above (amount of 0.10 g of composition per g of hair). The switch is kept at high humidity (above 85%) for 2 hours. Then, ten experts are asked to rate each hair switch in terms of frizz, clean feel, and greasy feel, based on a 5 point scale, 5 being the highest and 1 being the lowest rating. Acceptable values are:

- For frizz, less than 2 (lower number corresponds to less frizz);
- For no greasy feel less than 3, (lower number corresponds to less greasy feel), and
- For clean feel greater than 3 (higher number corresponds to cleaner feel).

Examples XIV-XVIII below are not within the scope of the invention.

Table 1 - Class I Compounds

| Formula Example | Control | XIV | XV | XVI | XVII | XVIII |
|---|---|---|---|---|---|---|
| **Raw Material** | | | | | | |
| Distilled Water | 50.0% | 49.5% | 49.5% | 49.5% | 49.5% | 49.5% |
| Ethanol | 50.0% | 49.5% | 49.5% | 49.5% | 49.5% | 49.5% |
| 5-Chlorosalicylic acid | 0% | 1% | 0% | 0% | 0% | 0% |
| Salicylic acid | 0% | 0% | 1% | 0% | 0% | 0% |
| 4-Hydroxybenzenesulphonic acid | 0% | 0% | 0% | 1% | 0% | 0% |
| 2,4-Dihydroxybenzoic acid | 0% | 0% | 0% | 0% | 1% | 0% |
| Terminal Amino Silicone | 0% | 0% | 0% | 0% | 0% | 1% |
| Composition pH adjusted to | 4.2 | 4.2 | 4.2 | 4.2 | 4.2 | 4.2 |
| Greasy Feel | 2 | 1 | 2 | 2 | 2 | 4 |
| Frizz | 4 | 2 | 1 | 2 | 2 | 3 |
| Clean Feel | 4 | 4 | 3 | 4 | 4 | 1 |

**Results of Hair Switch Rating from Class I Molecules (no within the scope of the invention):**

Molecules (5-chlorosalicylic acid, salicylic acid, 4-hydroxybenzenesulphonic acid, 2,4-dihydroxybenzoic acid) from Class I provide hair benefits. More specifically, Table 1 shows that hair treatments with 5-chlorosalicyclic acid, salicylic acid, 4-hydroxybenzenesulfonic acid and 2,4-dihydroxybenzoic acid provide frizz protection with clean feel and without greasy feel negative, as opposed to treatment with terminal aminosilicone, which provide some frizz benefit but with greasy feel negative and significantly less clean feel.

Examples XIX-XXIII are not within the scope of the invention.

Table 2 - Class II Compounds

| Formula Example | Control | XIX | XX | XXI | XXII | XXIII |
|---|---|---|---|---|---|---|
| **Raw Material** | | | | | | |
| Distilled Water | 50.0% | 49.5% | 49.5% | 49.5% | 49.5% | 49.5% |
| Ethanol | 50.0% | 49.5% | 49.5% | 49.5% | 49.5% | 49.5% |
| Isostearyl isostearate | 0% | 1% | 0% | 0% | 0% | 0% |
| 2-Hydroxyethyl salicylate | 0% | 0% | 1% | 0% | 0% | 0% |
| Octyl salicylate | 0% | 0% | 0% | 1% | 0% | 0% |
| 2-Hexyl-1-decanol | 0% | 0% | 0% | 0% | 1% | 0% |

(continued)

| Formula Example | Control | XIX | XX | XXI | XXII | XXIII |
|---|---|---|---|---|---|---|
| **Raw Material** | | | | | | |
| Terminal Amino Silicone | 0% | 0% | 0% | 0% | 0% | 1% |
| Composition pH adjusted to | 4.2 | 4.2 | 4.2 | 4.2 | 4.2 | 4.2 |
| Greasy Feel | 2 | 2 | 2 | 2 | 3 | 4 |
| Frizz | 4 | 2 | 2 | 1 | 1 | 3 |
| Clean Feel | 4 | 3 | 3 | 3 | 3 | 1 |

**Results of Hair Switch Rating from Class II Molecules:** Molecules (Isostearyl isostearate, 2-hydroxylethyl salicylate, octyl salicylate, 2-hexyl-1-decanol) from Class II provide hair benefits. More specifically, Table 2 shows that hair treatment with isostearyl isostearate, 2-hydroxyethyl salicylate, octyl salicylate, and 2-hexyl-1-decanol provide frizz protection with clean feel and without greasy feel negative, as opposed to treatment with terminal aminosilicone, which provide some frizz benefit but with greasy feel negative and significantly less clean feel.

Evaluation of Hair Friction

Leave-on formulation containing Moisture Control Material and Silicone oil shows improvement in dry feel compared to untreated hair. This is concluded by measurement of dry hair friction. For this evaluation, natural virgin brown hair switches (4.0 g) are washed with clarifying shampoo, and then treated with leave-on treatment of composition XXIV according to the protocol described above. Before the evaluation, the switches are air dried overnight in a controlled temperature and humidity room (22°C/50% RH). The friction force (grams) between the hair surface and a urethane pad along the hair is measured, with three measurements per switch using an Instron Tester instrument (Instron 5542, Instron, Inc, Canton, Mass., USA).
Example XXIV below is not within the scope of the invention.

Table 3 - Hair Friction

| Formula Example | XXIV | Control Hair - No Treatment |
|---|---|---|
| Raw Material | | |
| Distilled Water | 49.5% | |
| Ethanol | 49.5% | |
| 2,4 dihydroxybenzoic acid | 1% | |
| Silicone oil | 0% | |
| Composition pH adjusted to | 4.2 | |
| Average Force (g) | 40 | 55 |

As Table 3 indicates, treatment of hair with leave-on composition containing Moisture Control material and silicone oil results in reduced hair friction, which indicates improved dry feel.
It is known that organic hydrophobic molecules that are naturally present inside the hair (e.g. as part of Cell Membrane Complex lipids) contribute to its strength and integrity. It is also known that cosmetic treatments, such as oxidative coloring and permanent shaping result in reduction of the concentration of such hydrophobic material from hair. Thus, penetration of hydrophobic materials (e.g. Class II materials) inside the hair can contribute to lipid replenishment, which, at the same time, reduces water uptake to deliver moisture or frizz control benefit. Combination of different Class II materials e.g. benzyl alcohol, 2-hexyl-1-decanol, isostearyl isostearate, have multi-functionality of penetration, getting embedded into lipid of hair and also increasing the penetration of other hydrophobic materials like oleic resulting in further increase hydrophobicity of the hair interior.

**LEAVE ON TREATMENT COMPOSITION HAVING A GEL MATRIX**

The leave-on treatment composition comprises a gel matrix comprising (1) one or more high melting point fatty compounds, (2) a cationic surfactant system, and (3) an aqueous carrier as recited in claim 1.

## A. CATIONIC SURFACTANT SYSTEM

The gel matrix of the leave-on composition includes a cationic surfactant system. The cationic surfactant system can be one cationic surfactant or a mixture of two or more cationic surfactants. Preferably, the cationic surfactant is selected from mono-long alkyl quaternized ammonium salt, di-long alkyl quaternized ammonium salt, mono-long alkyl amidoamine salt or mixtures thereof. The cationic surfactant system is included in the composition at a level by weight of from 0.1% to 10%, from 0.5% to 8%, from 0.8 % to 5%, and from 1.0% to 4%.

The mono-long alkyl quaternized ammonium salt cationic surfactants useful herein are those having one long alkyl chain which has from 12 to 30 carbon atoms, preferably from 16 to 24 carbon atoms, more preferably 18 to 22 carbon atoms. The remaining groups attached to the nitrogen are independently selected from an alkyl group of from 1 to 4 carbon atoms or an alkoxy, polyoxyalkylene, alkylamido, hydroxyalkyl, aryl or alkylaryl group having up to 4 carbon atoms. The counterion is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g., those of 12 carbons, or higher, can be saturated or unsaturated. Nonlimiting examples of such mono-long alkyl quaternized ammonium salt cationic surfactants include: behenyl trimethyl ammonium salt.

The di-long alkyl quaternized ammonium salt cationic surfactants useful herein are those having two long alkyl chains of from 12 to 30 carbon atoms, more preferably from 16 to 24 carbon atoms, still more preferably from 16 to 22 carbon atoms or an aromatic, alkoxy, polyoxyalkylene, alkylamido, hydroxyalkyl, aryl or alkylaryl group having up to 30 carbon atoms. The remaining substituents on the nitrogen atom are selected from an aliphatic group of from 1 to 8 carbon atoms, preferably from 1 to 3 carbon atoms or an aromatic, alkoxy, polyoxyalkylene, alkylamido, hydroxyalkyl, aryl or alkylaryl group having up to 8 carbon atoms. The counterion is a salt forming anion selected from the group consisting of halides such as chloride and bromide, C1-C4 alkyl sulfate such as methosulfate and ethosulfate, and mixtures thereof. The aliphatic groups can contain, in addition to carbon and hydrogen atoms, ether linkages, and other groups such as amino groups. The longer chain aliphatic groups, e.g., those of 16 carbons, or higher, can be saturated or unsaturated. Nonlimiting examples of di-long alkyl cationic surfactants include dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride.

Mono-long alkyl amines are also suitable as cationic surfactants. Primary, secondary, and tertiary fatty amines are useful. Particularly useful are tertiary amido amines having an alkyl group of from 12 to 22 carbons. Exemplary tertiary amido amines include: stearamidopropyldimethylamine, stearamidopropyldiethylamine, stearamidoethyldiethyl-amine, stearamidoethyldimethylamine, palmitamidopropyldimethylamine, palmitamidopropyldiethylamine, palmitami-doethyldiethylamine, palmitamidoethyldimethylamine, behenamidopropyldimethylamine, behenamidopropyldiethyl-amine, behenamidoethyldiethylamine, behenamidoethyldimethylamine, arachidamidopropyldimethylamine, arachidam-idopropyldiethylamine, arachidamidoethyldiethylamine, arachidamidoethyldimethylamine, diethylaminoethylsteara-mide.

## HIGH MELTING POINT FATTY COMPOUND

The high melting point fatty compound is included in the composition at a level of from 0.1% to 20%, by weight of the composition, in view of providing the benefits of the composition. The high melting point fatty compound useful herein have a melting point of 25°C or higher, preferably 40°C or higher, more preferably 45°C or higher, still more preferably 50°C or higher. The high melting point fatty compound can be used as a single compound or as a blend or mixture of at least two high melting point fatty compounds. When used as such blend or mixture, the above melting point means the melting point of the blend or mixture.

The high melting point fatty compound useful herein is selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature.

Among a variety of high melting point fatty compounds, fatty alcohols are suitable for use in the conditioner composition. The fatty alcohols useful herein are those having from 14 to 30 carbon atoms, from 16 to 22 carbon atoms. These fatty alcohols are saturated and can be straight or branched chain alcohols. Suitable fatty alcohols include, for example, cetyl alcohol, stearyl alcohol, behenyl alcohol, and mixtures thereof.

## AQUEOUS CARRIER

The conditioner gel matrix of the leave-on treatment composition includes an aqueous carrier which can be water or a mixture of water and water-miscible solvents. The gel matric comprises at least 20% of an aqueous carrier, by weight of said aqueous hair leave-on composition.

### *Leave on Composition Containing Gel Matrix Preparation:*

A non-limiting example of leave on formulation compositions can be prepared by any conventional method well known in the art containing gel matrix. The cationic surfactants and the fatty alcohols are mixed together and heated to from 66°C to 85°C to form an oil phase. Separately, the disodium EDTA, the Methylchloroisothiazolinone (preservative) and the water are mixed and heated to from 20°C to 48°C to form an aqueous phase. The oil phase is mixed into the water phase under high shear to form the gel matrix. The remaining of the components are added into the gel matrix with agitation. Then, the composition is cooled down to room temperature.

Table 4: Moisture control materials in leave on formulation containing Gel Matrix Example XIVa below is not within the scope of the invention.

| Formula Example | Active% | XIVa | XVa |
|---|---|---|---|
| Raw Material | | (wt. / wt.)% | (wt. / wt.)% |
| Hydroxyethyl cellulose[1] | 80 | 0.400 | 0.400 |
| Cetyl Alcohol[2] | 90 | 0.575 | 0.575 |
| Stearyl Alcohol[3] | 97 | 0.383 | 0.383 |
| Benzyl Alcohol[4] | 99 | 0.400 | 0.400 |
| Disodium EDTA, Dihydrate[5] | 99 | 0.127 | 0.127 |
| Glyceryl monostearate (PoloxWSR N-10)[6] | 1.5 | 0.299 | 0.299 |
| Terminal Amino Silicone[7] | 90-100 | 2 | 2 |
| Perfume | | 0.550 | 0.550 |
| Salicylic acid[8] | 99.5 | 0 | 2.0 |
| Isostearyl Isostearate[11] | 100 | 0 | 1.0 |
| 2-hexyldecanol[12] | 97 | 0 | 5.0 |
| Purified Water | | Q.S. | Q.S. |
| % Water Reduction versus XIV (control) at dose of 0.1g of composition for 1g of hair | | - | 4 |
| pH | | 5.2 | 5.2 |
| % Frizz Reduction | | | 20 |

[1]. Natrosol™ hydroxyethylcellulose Supplied by Ashland (Kentucky, US)

[2]. Supplied by P&G Chemicals

[3]. Supplied by P&G Chemicals

[4]. Supplied by Ineos Maastricht BV (Maastricht NL)

[5]. Trilon BD Powder supplied by BASF SE (Ludwigshafen, DE)

[6]. POLYOX™ WSR N-10 (Glyceryl monostearate) supplied by Dow chemicals (Michigan US)

[7]. Y-14945 supplied by Momentive Performance Materials

[8]. Supplied by API Corporation

[9]. Supplied by Sigma Aldrich

[10]. Supplied by Sigma Aldrich

[11]. Crodamol ISIS supplied by Croda

[12]. Isofol 16 supplied by Sasol (Brunsbuettel, DE)

**Results:** Hair Switches treated with leave on treatment of example XVa, using the leave on hair treatment protocol described in page 12, shows % water reduction by DVS method of 4% vs hair treated with comparative example XIVa control.

**Rheology Modifier**

The leave-on hair care composition may comprise a rheology modifier to increase the substantivity and stability of the composition. Any suitable rheology modifier can be used. The leave-on hair care composition may comprise from 0.05% to 10% of a rheology modifier, or, from 0.1% to 10% of a rheology modifier, or, from 0.5% to 2 % of a rheology modifier, or, from 0.7% to 2% of a rheology modifier, or from 1% to 1.5% of a rheology modifier. The rheology modifier may be a polyacrylamide thickener. The rheology modifier may be a polymeric rheology modifier.

The leave-on hair care composition may comprise rheology modifiers that are homopolymers based on acrylic acid, methacrylic acid or other related derivatives, non-limiting examples include polyacrylate, polymethacrylate, polyethylacrylate, and polyacrylamide.

The rheology modifiers may be alkali swellable and hydrophobically-modified alkali swellable acrylic copolymers or methacrylate copolymers non-limiting examples include acrylic acid/acrylonitrogen copolymer, acrylates/steareth-20 itaconate copolymer, acrylates/ceteth-20 itaconate copolymer, acrylates/aminoacrylates copolymer, acrylates/steareth-20 methacrylate copolymer, acrylates/beheneth-25 methacrylate copolymer, acrylates/steareth-20 methacrylate crosspolymer, acrylates/vinylneodecanoate crosspolymer, and acrylates/C10-C30 alkyl acrylate crosspolymer.

The rheology modifiers may be crosslinked acrylic polymers, a non-limiting example includes carbomers.

The rheology modifiers may be alginic acid-based materials; non-limiting examples include sodium alginate, and alginic acid propylene glycol esters.

The rheology modifier may be an associative polymeric thickeners, non-limiting examples include: Hydrophobically modified cellulose derivatives; Hydrophobically modified alkoxylated urethane polymers, nonlimiting example include PEG-150/decyl alcohol/SMDI copolymer, PEG-150/stearyl alcohol/SMDI copolymer, polyurethane-39; Hydrophobically modified, alkali swellable emulsions, non-limiting examples include hydrophobically modified polyacrylates, hydrophobically modified polyacrylic acids, and hydrophobically modified polyacrylamides; hydrophobically modified polyethers wherein these materials may have a hydrophobe that can be selected from cetyl, stearyl, oleayl, and combinations thereof, and a hydrophilic portion of repeating ethylene oxide groups with repeat units from 10-300, or from 30-200, or from 40-150. Non-limiting examples of this class include PEG-120-methylglucose dioleate, PEG-(40 or 60) sorbitan tetraoleate, PEG -150 pentaerythrityl tetrastearate, PEG-55 propylene glycol oleate, PEG-150 distearate.

The rheology modifier may be cellulose and derivatives; nonlimiting examples include microcrystalline cellulose, carboxymethylcelluloses, hydroxyethylcellulose, hydroxypropylcellulose, hydroxypropylmethylcellulose, methylcellulose, ethylcellulose, nitro cellulose, cellulose sulfate, cellulose powder, and hydrophobically modified celluloses.

The rheology modifier may be a guar and guar derivatives; nonlimiting examples include hydroxypropyl guar, and hydroxypropyl guar hydroxypropyl trimonium chloride.

The rheology modifier may be polyethylene oxide, polypropylene oxide, and POE-PPO copolymers.

The rheology modifier may be polyvinylpyrrolidone, crosslinked polyvinylpyrrolidone and derivatives. The rheology modifier may be polyvinyalcohol and derivatives.

The rheology modifier may be polyethyleneimine and derivatives.

The rheology modifier may be silicas; nonlimiting examples include fumed silica, precipitated silica, and silicone-surface treated silica.

The rheology modifier may be water-swellable clays non-limiting examples include laponite, bentolite, montmorilonite, smectite, and hectonite.

The rheology modifier may be gums nonlimiting examples include xanthan gum, guar gum, hydroxypropyl guar gum, Arabia gum, tragacanth, galactan, carob gum, karaya gum, and locust bean gum.

The rheology modifier may be, dibenzylidene sorbitol, karaggenan, pectin, agar, quince seed (Cydonia oblonga Mill), starch (from rice, corn, potato, wheat, etc), starch-derivatives (e.g. carboxymethyl starch, methylhydroxypropyl starch), algae extracts, dextran, succinoglucan, and pulleran,

Non-limiting examples of rheology modifiers include acrylamide/ammonium acrylate copolymer (and)polyisobutene (and) polysorbate 20, acrylamide/sodium acryloyldimethyl taurate copolymer/ isohexadecane/ polysorbate 80, acrylates copolymer; acrylates/beheneth-25 methacrylate copolymer, acrylates/C10-C30 alkyl acrylate crosspolymer, acrylates/steareth-20 itaconate copolymer, ammonium polyacrylate/Isohexadecane/PEG-40 castor oil, C12-16 alkyl PEG-2 hydroxypropylhydroxyethyl ethylcellulose (HM-EHEC), carbomer, crosslinked polyvinylpyrrolidone (PVP), dibenzylidene sorbitol, hydroxyethyl ethylcellulose (EHEC), hydroxypropyl methylcellulose (HPMC), hydroxypropyl methylcellulose (HPMC), hydroxypropylcellulose (HPC), methylcellulose (MC), methylhydroxyethyl cellulose (MEHEC), PEG-150/decyl alcohol/SMDI copolymer, PEG-150/stearyl alcohol/SMDI copolymer, polyacrylamide/C13-14 isoparaffin/laureth-7; polyacrylate 13/polyisobutene/polysorbate 20; polyacrylate crosspolymer-6, polyamide-3; polyquatemium-37 (and) hydrogenated polydecene (and) trideceth-6, polyurethane-39, sodium acrylate/acryloyldimethyltaurate/dimethylacrylamide, crosspolymer (and) isohexadecane (and) polysorbate 60; sodium polyacrylate. Exemplary commercially-available rheology modifiers include ACULYN™ 28, Klucel M CS, Klucel H CS, Klucel G CS, SYLVACLEAR AF1900V, SYLVACLEAR PA1200V, Benecel E10M, Benecel K35M, Optasense RMC70, ACULYN™33, ACULYN™46, ACULYN™22, ACULYN™44, Carbopol Ultrez 20, Carbopol Ultrez 21, Carbopol Ultrez 10, Carbopol Ulterez 30, Carbopol 1342, Sepigel™ 305, Simulgel™600, Sepimax Zen, and combinations thereof.

## Carrier

The leave-on hair care compositions further includes at least 20 weight percent of an aqueous carrier. The aqueous carrier may be prepared from demineralized or distilled water, for example. The carrier may comprise water, organic solvents (miscible or non-miscible with water), silicone solvents or a mixture thereof. A volatile carrier may include water or a mixture of water and organic solvents. The solvents may be dermatologically acceptable. The carrier may comprise water with minimal or no significant concentrations of organic solvent, except as otherwise incidentally incorporated into the composition as minor ingredients of other components. Water, organic and silicone solvents that have boiling points below or equal to 250°C may be volatile solvents and volatile carriers. Solvents with boiling points above 250°C may be considered non-volatile.

Non-limiting examples of a carrier may include water and solutions or mixtures of water with lower alkyl alcohols and/or polyhydric alcohols. Examples of lower alkyl alcohols are monohydric alcohols having 1 to 6 carbons such as ethanol, methanol, propanol, isopropanol, butanol, pentanol, and hexanol. Examples of polyhydric alcohols are glycols, such as dipropylene glycol, propylene glycol, butylene glycol, 1,4-butanediol, 3-allyloxy-1,2-propanediol, 1,2-hexanediol, 1,6-hexanediol, 1,3-butanediol, 1,3-propanediol, 2,2'-thiodiethanol, glycerin and other diols.

Other non-limiting examples of organic solvents include polyglycols such as polypropylene glycol, polyethylene glycol, mixture of butylene glycol, polypropylene glycol, polyethylene glycol and ethers, such as dipropylene glycol n-butyl ether, sugars, and sugar derivatives.

Penetration of Moisture Control Material inside the hair

Compositions can comprise of glycols, polyglycols, urea, ethers or mixture thereof. These materials increase penetration of moisture control actives such as salicylic acid, 5-chloro salicylic acid, improving their performance. Propylene glycol, butylene glycol and other glycols, increase penetration of 5-chlorosalicylic acid inside hair as it acts as carrier for the actives to penetrate further. As active penetration increases, there is an increase in efficacy of the active, i.e. there is increase in % water reduction as shown below in Table 5. Table 5 shows the amount of 5-chlorosalicylic acid that penetrates inside oxidatively damaged hair after hair treatment with two different compositions. It also shows the % water reduction observed after the treatment versus treatment with control leave-on treatment compositions. These results demonstrate that 5-chlorosalicylic acid penetrates 4 times more in the presence of propylene glycol and there is an increase in % water reduction as measured by DVS of approximate 4 times more than without propylene glycol. A further non-limiting example of a material that enhances the penetration of moisture control material is 2-hydroxyethyl urea. Leave on treatment composition that contain 2% of 2-hydroxyethyl urea increases the penetration of salicylic acid inside hair by 14% compared to the corresponding composition that does not contain 2-hydroxyethyl urea (see example XXVII and XXVIII).

| Table 5 - Enhancing of hair penetration of Moisture Control Material in oxidatively damaged (bleached) Caucasian hair | | | | | |
|---|---|---|---|---|---|
| Formula Example | Control | XXV | XXVI | XXVII | XXVIII |
| Raw Material | | | | | |
| Distilled Water | 50.0% | 48.93% | 43.9% | 48.93% | 48.00% |
| Ethanol | 50.0% | 48.93% | 43.9% | 48.93% | 48.00% |
| 5-Chlorosalicylic acid | 0.0% | 2.0% | 2.0% | 0.0% | 0.0% |
| 2,4-Dihydroxybenzoic acid | 0.0% | 0.15% | 0.15% | 0.0% | 0.0% |
| Propylene glycol | 0.0% | 0% | 10% | 0.0% | 0.0% |
| Composition pH adjusted to | 4.2 | 4.2 | 4.2 | 4.2 | 4.2 |
| 2-hydroxyethyl urea | 0.0% | 0.0% | 0.0% | 0% | 2.0% |

(continued)

| Table 5 - Enhancing of hair penetration of Moisture Control Material in oxidatively damaged (bleached) Caucasian hair | | | | | |
|---|---|---|---|---|---|
| Formula Example | Control | XXV | XXVI | XXVII | XXVIII |
| Raw Material | | | | | |
| Salicylic acid | 0.0% | 0.0% | 0.0% | 2.0% | 2.0% |
| % Water Reduction versus control treatment | - | 0.67% | 3% | - | - |
| Amount of 5-chlorosalicylic acid inside the hair (mg/g of hair) | - | 1 | 3.97 | - | - |
| Amount of Salicylic acid inside hair (mg/g of hair) after 5 cycles | - | - | - | 4.7 | 5.6 |

Examples XXV-XXVIII just above are not within the scope of the invention.

The penetration amount of 5-chlorosalicylic acid is determined using the following protocol. Each hair tress is extracted 3 times with 0.1% TFA (Trifluoroacetic acid) in methanol and the individual extracts are analyzed separately using HPLC method.

In addition to the increase of the penetration amount of the moisture control material, the presence of glycol in the composition prevents the crystallization of part of the moisture control material in the surface of the hair. Such crystallization causes a non-smooth, negative hair feel, which may be perceived by consumers as hair damage or lack of conditioning.

It has been observed that the presence of propylene glycol may provide penetration enhancement for Class I and Class II materials.

**Silicones**

The conditioning agent of the compositions can be a silicone conditioning agent. The silicone conditioning agent may comprise volatile silicone, non-volatile silicone, or combinations thereof. The concentration of the silicone conditioning agent typically ranges from 0.01% to 10%, by weight of the composition, from 0.1% to 8%, from 0.1% to 5%, and/or from 0.2% to 3%. Non-limiting examples of suitable silicone conditioning agents, and optional suspending agents for the silicone, are described in U.S. Reissue Pat. No. 34,584, U.S. Pat. No. 5,104,646, and U.S. Pat. No. 5,106,609. The silicone conditioning agents for use in the compositions can have a viscosity, as measured at 25° C, from 20 to 2,000,000 centistokes ("csk"), from 1,000 to 1,800,000 csk, from 50,000 to 1,500,000 csk, and/or from 100,000 to 1,500,000 csk.

The dispersed silicone conditioning agent particles typically have a volume average particle diameter ranging from 0.01 micrometer to 50 micrometer. For small particle application to hair, the volume average particle diameters typically range from 0.01 micrometer to 4 micrometer, from 0.01 micrometer to 2 micrometer, from 0.01 micrometer to 0.5 micrometer. For larger particle application to hair, the volume average particle diameters typically range from 5 micrometer to 125 micrometer, from 10 micrometer to 90 micrometer, from 15 micrometer to 70 micrometer, and/or from 20 micrometer to 50 micrometer.

Additional material on silicones including sections discussing silicone fluids, gums, and resins, as well as manufacture of silicones, are found in Encyclopedia of Polymer Science and Engineering, vol. 15, 2d ed., pp 204-308, John Wiley & Sons, Inc. (1989).

Silicone emulsions suitable for use herein may include, but are not limited to, emulsions of insoluble polysiloxanes prepared in accordance with the descriptions provided in U.S. Patent No. 4,476,282 and U.S. Patent Application Publication No. 2007/0276087. Accordingly, suitable insoluble polysiloxanes include polysiloxanes such as alpha, omega hydroxy-terminated polysiloxanes or alpha, omega alkoxy-terminated polysiloxanes having a molecular weight within the range from 50,000 to 500,000 g/mol. The insoluble polysiloxane can have an average molecular weight within the range from 50,000 to 500,000 g/mol. For example, the insoluble polysiloxane may have an average molecular weight within the range from 60,000 to 400,000; from 75,000 to 300,000; from 100,000 to 200,000; or the average molecular weight may be 150,000 g/mol. The insoluble polysiloxane can have an average particle size within the range from 30 nm to 10 micron. The average particle size may be within the range from 40 nm to 5 micron, from 50nm to 1micron, from 75 nm to 500 nm, or 100 nm, for example.

The average molecular weight of the insoluble polysiloxane, the viscosity of the silicone emulsion, and the size of the particle comprising the insoluble polysiloxane are determined by methods commonly used by those skilled in the art, such as the methods disclosed in Smith, A. L. The Analytical Chemistry of Silicones, John Wiley & Sons, Inc.: New York, 1991. For example, the viscosity of the silicone emulsion can be measured at 30°C with a Brookfield viscometer with spindle 6 at 2.5 rpm. The silicone emulsion may further include an additional emulsifier together with the anionic surfactant,

Other classes of silicones suitable for use in the compositions may include but are not limited to: i) silicone fluids, including but not limited to, silicone oils, which are flowable materials having viscosity less than 1,000,000 csk as measured at 25°C; ii) aminosilicones, which contain at least one primary, secondary or tertiary amine; iii) cationic silicones, which contain at least one quaternary ammonium functional group; iv) silicone gums; which include materials having viscosity greater or equal to 1,000,000 csk as measured at 25°C; v) silicone resins, which include highly cross-linked polymeric siloxane systems; vi) high refractive index silicones, having refractive index of at least 1.46, and vii) mixtures thereof.

**Organic Conditioning Materials**

The conditioning agent of the compositions may also comprise at least one organic conditioning material such as oil or wax, either alone or in combination with other conditioning agents, such as the silicones described above. The organic material can be non-polymeric, oligomeric or polymeric. It may be in the form of oil or wax and may be added in the formulation neat or in a pre-emulsified form. Some non-limiting examples of organic conditioning materials include, but are not limited to: i) hydrocarbon oils; ii) polyolefins, iii) fatty esters, iv) fluorinated conditioning compounds, v) fatty alcohols, vi) alkyl glucosides and alkyl glucoside derivatives; vii) quaternary ammonium compounds; viii) polyethylene glycols and polypropylene glycols having a molecular weight of up to 2,000,000 including those with CTFA names PEG-200, PEG-400, PEG-600, PEG-1000, PEG-2M, PEG-7M, PEG-14M, PEG-45M and mixtures thereof.

**Hair Health Actives**

A scalp health active may be added to provide scalp benefits. This group of materials is varied and provides a wide range of benefits including anti-dandruff, anti-fungal, anti-microbial, moisturization, barrier improvement, and anti-oxidant, anti-itch, and sensates. Such skin health actives include but are not limited to: zinc pyrithione, climbazole, octopirox, vitamin E and F, salicylic acid, glycols, glycolic acid, PCA, PEGs, erythritol, glycerin, lactates, hyaluronates, allantoin and other ureas, betaines, sorbitol, glutamates, xylitols, menthol, menthyl lactate, isocyclomone, benzyl alcohol, and natural extracts/oils including peppermint, spearmint, argan, jojoba and aloe.

**Anti- Dandruff Actives**

The compositions may contain anti-dandruff agents. When present in these compositions, the anti-dandruff agent is typically included in an amount of 0.01 wt. % to 5 wt. %, based on the total weight of the personal care composition. In these compositions, the anti-dandruff particulate should be physically and chemically compatible with other ingredients of the composition, and should not otherwise unduly impair product stability, aesthetics, or performance.

Anti-dandruff agents suitable for use in personal care compositions include pyridinethione salts, azoles (e.g.,ketoconazole, econazole, and elubiol), selenium sulfide, particulate sulfur, salicylic acid, and mixtures thereof. A typical anti-dandruff agent is pyridinethione salt. Personal care compositions can also include a zinc-containing layered material. An example of a zinc-containing layered material can include zinc carbonate materials. Of these, zinc carbonate and pyridinethione salts (particularly zinc pyridinethione or "ZPT) are common in the composition, and often present together.

In addition to the anti-dandruff active, compositions may also include one or more anti-fungal or anti-microbial actives in addition to the metal pyrithione salt actives. Suitable anti-microbial actives include coal tar, sulfur, charcoal, whitfield's ointment, castellani's paint, aluminum chloride, gentian violet, octopirox (piroctone olamine), ciclopirox olamine, undecylenic acid and it's metal salts, US 2011/0305778 A1 Dec. 15, 2011 potassium permanganate, selenium sulphide, sodium thiosulfate, propylene glycol, oil of bitter orange, urea preparations, griseofulvin, 8-Hydroxyquinoline ciloquinol, thiobendazole, thiocarbamates, haloprogin, polyenes, hydroxypyridone, morpholine, benzylamine, allylamines (such as terbinafine), tea tree oil, clove leaf oil, coriander, palmarosa, berberine, thyme red, cinnamon oil, cinnamic aldehyde, citronellic acid, hinokitol, ichthyol pale, Sensiva SC-50, Elestab HP- 100, azelaic acid, lyticase, iodopropynyl butylcarbamate (IPBC), isothiazalinones such as octyl isothiazalinone and azoles, and combinations thereof. Typical anti-microbials include itraconazole, ketoconazole, selenium sulphide and coal tar.

i. Azoles

Azole anti-microbials include imidazoles such as benzimidazole, benzothiazole, bifonazole, butaconazole nitrate, climbazole, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, tioconazole, thiazole, and triazoles such as terconazole and itraconazole, and combinations thereof. When present in the composition, the azole anti-microbial active is included in an amount from 0.01 wt. % to 5 wt. %, typically from 0.1 wt. % to 3 wt. %, and commonly from 0.3 wt. % to 2 wt. %, based on the total weight of the personal care product. Especially common for use herein is ketoconazole.

ii. Selenium Sulfide

Selenium sulfide is a particulate anti-dandruff agent suitable for use in anti-microbial personal care compositions, effective concentrations of which range from 0.1 wt. % to 4 wt. %, based on the total weight of the personal care product, typically from 0.3 wt. % to 2.5 wt. %, commonly from 0.5 wt. % to 1.5 wt. %. Selenium sulfide is generally regarded as a compound having one mole of selenium and two moles of sulfur, although it may also be a cyclic structure that conforms to the general formula Se S y, wherein x+y=8. Average particle diameters for the selenium sulfide are typically less than 15 $\mu$m, as measured by forward laser light scattering device (e.g. Malvern 3600 instrument), typically less than 10 $\mu$m. Selenium sulfide compounds are described, for example, in U.S. Pat. No. 2,694,668; U.S. Pat. No. 3,152,046; U.S. Pat. No. 4,089,945; and U.S. Pat. No. 4,885,107.

iii. Sulfur

Sulfur may also be used as a particulate anti-microbial/ anti-dandruff agent in anti-microbial personal care compositions. Effective concentrations of the particulate sulfur are typically from 1 wt. % to 4 wt. %, based on the total weight of the personal care product, typically from 2 wt. % to 4 wt. %.

iv. Keratolytic Agents

The personal care composition can further include one or more keratolytic agents such as salicylic acid. The personal care composition may also include a combination of anti-microbial actives. Such combinations may include octopirox and zinc pyrithione, pine tar and sulfur combinations, salicylic acid and zinc pyrithione combinations, salicylic acid and elubiol combinations, zinc pyrithione and elubiol combinations, octopirox and climbazole combinations, and salicylic acid and octopirox combinations and mixtures thereof.

II. Zinc-Containing Material, Including Zinc Carbonate

Compositions may include a zinc-containing layered material. Those compositions can include 0.001 wt. % to 10 wt. % of the zinc-containing layered material based on the total weight of the personal care composition. A personal care composition can include a zinc-containing layered material from 0.01 wt. % to 7 wt. % based on the total weight of the personal care composition. A personal care composition can include a zinc-containing layered material from 0.1 wt. % to 5 wt. %, based on the total weight of the composition. Suitable zinc-containing layered materials include those described below, including zinc carbonate materials, which are presently preferred:
Zinc-containing layered structures are those with crystal growth primarily occurring in two dimensions. It is conventional to describe layer structures as not only those in which all the atoms are incorporated in well-defined layers, but also those in which there are ions or molecules between the layers, called gallery ions (A. F. Wells "Structural Inorganic Chemistry" Clarendon Press, 1975) Zinc-containing layered materials (ZLM's) may have zinc incorporated in the layers and/or be components of the gallery ions.

Many ZLM's occur naturally as minerals. Common examples include hydrozincite (zinc carbonate hydroxide), basic zinc carbonate, aurichalcite (zinc copper carbonate hydroxide), rosasite (copper zinc carbonate hydroxide) and many related minerals that are zinc-containing. Natural ZLM's can also occur wherein anionic layer species such as clay-type minerals (e.g., phyllosilicates) contain ion-exchanged zinc gallery ions. All of these natural materials can also be obtained synthetically or formed in situ in a composition or during a production process.

Another common class of ZLM's, which are often, but not always, synthetic, is layered doubly hydroxides, which are generally represented by the formula $[M^{2+}_{1-x}M^{3+}_{x}(OH)_2]^{x+} A^{m-}_{x/m} \cdot nH_2O$ and some or all of the divalent ions ($M^{2+}$) would be represented as zinc ions (Crepaldi, E L, Pava, P C, Tronto, J, Valim, J B J. Colloid Interfac. Sci. 2002, 248, 429-42).

Yet another class of ZLM's can be prepared called hydroxy double salts (Morioka, H., Tagaya, H., Karasu, M, Kadokawa, J, Chiba, K Inorg. Chem. 1999, 38, 4211-6). Hydroxy double salts can be represented by the general formula $[M^{2+}_{1-x}M^{2+}_{1+x}(OH)_{3(1-y)}]^{+} A^{n-}_{(1=3y)/n} \cdot nH_2O$ where the two metal ion may be different; if they are the same and represented by zinc, the formula simplifies to $[Zn_{1+x}(OH)_2]^{2x+} 2x A^{-} \cdot nH_2O$. This latter formula represents (where x=0.4) common materials such as zinc hydroxychloride and zinc hydroxynitrate. These are related to hydrozincite as well wherein a divalent anion replace the monovalent anion. These materials can also be formed in situ in a composition or in or during a production process.

These classes of ZLM's represent relatively common examples of the general category and are not intended to be limiting as to the broader scope of materials which fit this definition.

Commercially available sources of basic zinc carbonate include Zinc Carbonate Basic (Cater Chemicals: Bensenville, Ill., USA), Zinc Carbonate (Shepherd Chemicals: Norwood, Ohio, USA), Zinc Carbonate (CPS Union US 2011/0305778 A1 Dec. 15, 2011 Ld Corp.: New York, N.Y., USA), Zinc Carbonate (Elementis Pigments: Durham, UK), and Zinc Carbonate AC (Bruggemann Chemical: Newtown Square, Pa., USA).

Basic zinc carbonate, which also maybe referred to commercially as "Zinc Carbonate" or "Zinc Carbonate Basic" or "Zinc Hydroxy Carbonate", is a synthetic version consisting of materials similar to naturally occurring hydrozincite. The idealized stoichiometry is represented by $Zn_5(OH)_6(CO_3)_2$ but the actual stoichiometric ratios can vary slightly and other impurities may be incorporated in the crystal lattice

Anti-dandruff efficacy can be dramatically increased in topical compositions by the combination of an anti-dandruff agent with an effective amount of a zinc-containing layered material, wherein the zinc-containing layered material has a specified zinc lability within a surfactant system. Zinc lability is a measure of the chemical availability of zinc ion. Soluble zinc salts that do not complex with other species in solution have a relative zinc lability, by definition, of 100%. The use of partially soluble forms of zinc salts and/or incorporation in a matrix with potential complexants generally lowers the zinc lability substantially below the defined 100% maximum.

Labile zinc is maintained by choice of an effective zinc-containing layered material or formation of an effective zinc-containing layered material in-situ by known methods.

Anti-dandruff efficacy can be dramatically increased in topical compositions by the use of polyvalent metal salts of pyrithione, such as zinc pyrithione, in combination with zinc-containing layered materials. Therefore, personal care compositions can include those containing both anti-dandruff agents and zinc-containing layered materials for topical application to provide improved benefits to the skin and scalp (e.g., improved antidandruff efficacy).

Optional Ingredients

The compositions can also additionally comprise any suitable optional ingredients as desired. For example, the composition can optionally include other active or inactive ingredients.

The compositions may include other common hair ingredients such as other anti-dandruff actives, minoxidil, conditioning agents, and other suitable materials. The CTFA Cosmetic Ingredient Handbook, Tenth Edition (published by the Cosmetic, Toiletry, and Fragrance Association, Inc., Washington, D.C.) (2004) (hereinafter "CTFA"), describes a wide variety of nonlimiting materials that can be added to the composition herein. Examples of these ingredient classes include, but are not limited to: abrasives, absorbents, aesthetic components such as fragrances, pigments, colorings/colorants, essential oils, skin sensates, astringents, etc. (e.g., clove oil, menthol, camphor, eucalyptus oil, eugenol, menthyl lactate, witch hazel distillate), anti-acne agents, anti-caking agents, antifoaming agents, antimicrobial agents (e.g., iodopropyl butylcarbamate), antioxidants, binders, biological additives, buffering agents, bulking agents, chelating agents, chemical additives, colorants, cosmetic astringents, cosmetic biocides, denaturants, drug astringents, external analgesics, film formers or materials, e.g., polymers, for aiding the film-forming properties and substantivity of the composition (e.g., copolymer of eicosene and vinyl pyrrolidone), opacifying agents, pH adjusters, propellants, reducing agents, sequestrants, rheology modifiers, hair conditioning agents, and surfactants.

The formulations o may be present in typical hair care compositions. They may be in the form of solutions, dispersion, emulsions, powders, talcs, encapsulated, spheres, spongers, solid dosage forms, foams, and other delivery mechanisms. The composition may be hair tonics, leave-on hair products such as conditioners, treatment, and styling products, and any other form that may be applied to the hair.

**Claims**

1. An aqueous hair leave-on composition for hair frizz reduction comprising from 0.15% to 12.0% of a moisture control material or mixture of moisture control materials by weight of the aqueous hair leave-on composition, wherein the moisture control material comprises 2-Hexyl-1-decanol in combination with Salicylic acid; wherein the leave-on composition further comprises a gel matrix comprising:

   i.) from 0.1% to 20% of one or more high melting point fatty compounds, by weight of said aqueous hair leave-on composition, wherein the one or more high melting point fatty compounds have a melting point of 25°C or higher;
   ii.) from 0.1% to 10% a cationic surfactant system, by weight of said aqueous hair leave-on composition; and

iii.) at least 20% of an aqueous carrier, by weight of said aqueous hair leave-on composition.

2. The aqueous hair leave-on composition according to claim 1, wherein the concentration of the Moisture Control Material or the concentration of the mixture of Moisture Control Material is from 0.2% to 5%, preferably from 0.5% to 4%, more preferably from 1.02% to 3.0%, by weight of the aqueous hair leave-on composition.

3. The aqueous hair leave-on composition according to any preceding claims wherein the composition further comprises isostearyl isostearate.

4. The aqueous hair leave-on composition according to any preceding claims wherein the composition further comprises propylene glycol.

5. The aqueous hair leave-on composition according to any preceding claims wherein the composition further comprises 2-hydroxyethyl urea.

6. The aqueous hair leave-on composition according to any preceding claims comprising 0.5% to 2% Salicylic acid in combination with 0.2% to 10% of 2-hydroxyethyl urea.

7. The aqueous hair leave-on composition according to any preceding claims wherein the composition further comprises materials selected from the group consisting of silicones, conditioning materials , solvents, rheology modifier, thickeners, hair health actives, anti-dandruff actives, anti-oxidants, pigments, abrasives, absorbents, biological actives, buffering agents, chelating agents, opacifying agents, pH adjusters and mixtures thereof.

8. The aqueous hair leave-on composition according to any preceding claims wherein the composition further comprises a cationic surfactant system.

9. The aqueous hair leave-on composition according to any preceding claims wherein the composition further comprises a non-ionic surfactant system.

10. Use of an aqueous hair leave-on composition for reducing hair frizz, wherein the aqueous hair leave-on composition comprises from 0.15% to 12.0% of a moisture control material or mixture of moisture control materials by weight of the aqueous hair leave-on composition, wherein the moisture control material comprises 2-Hexyl-1-decanol in combination with Salicylic acid, wherein the leave-on composition further comprises a gel matrix comprising:

   i.) from 0.1% to 20% of one or more high melting point fatty compounds, by weight of said aqueous hair leave-on composition, wherein the one or more high melting point fatty compounds have a melting point of 25°C or higher;
   ii.) from 0.1% to 10% a cationic surfactant system, by weight of said aqueous hair leave-on composition; and
   iii.) at least 20% of an aqueous carrier, by weight of said aqueous hair leave-on composition.

**Patentansprüche**

1. Wässrige Zusammensetzung, die zum Belassen auf dem Haar bestimmt ist, für eine Haarkrausreduktion, umfassend von zu 0,15 Gew.-% bis 12,0 Gew.-% der wässrigen Zusammensetzung, die zum Belassen auf dem Haar bestimmt ist, ein Feuchtigkeitsregulierungsmaterial oder eine Mischung von Feuchtigkeitsregulierungsmaterialien, wobei das Feuchtigkeitsregulierungsmaterial 2-Hexyl-1-decanol in Kombination mit einer Salicylsäure umfasst; wobei die Zusammensetzung, die zum Belassen auf dem Haar bestimmt ist, ferner eine Gelmatrix umfasst, umfassend:

   i.) von zu 0,1 Gew.-% bis 20 Gew.-% der wässrigen Zusammensetzung, die zum Belassen auf dem Haar bestimmt ist, eine oder mehrere Fettverbindungen mit hohem Schmelzpunkt, wobei die eine oder die mehreren Fettverbindungen mit hohem Schmelzpunkt einen Schmelzpunkt von 25 °C oder höher aufweisen;
   ii.) von zu 0,1 Gew.-% bis 10 Gew.-% der Zusammensetzung, die zum Belassen auf dem Haar bestimmt ist, ein kationenaktives Tensidsystem; und
   iii.) wenigstens zu 20 Gew.-% der Zusammensetzung, die zum Belassen auf dem Haar bestimmt ist, einen wässrigen Träger.

2. Wässrige Zusammensetzung, die zum Belassen auf dem Haar bestimmt ist, nach Anspruch 1, wobei die Konzentration des Feuchtigkeitsregulierungsmaterials oder die Konzentration der Mischung des Feuchtigkeitsregulierungsmaterials von zu 0,2 Gew.-% bis 5 Gew.-%, vorzugsweise von zu 0,5 Gew.-% bis 4 Gew.-%, mehr bevorzugt von zu 1,02 Gew.-% bis 3,0 Gew.-% der Zusammensetzung, die zum Belassen auf dem Haar bestimmt ist, beträgt.

3. Wässrige Zusammensetzung, die zum Belassen auf dem Haar bestimmt ist, nach einem der vorstehenden Ansprüche, wobei die Zusammensetzung ferner Isostearylisostearat umfasst.

4. Wässrige Zusammensetzung, die zum Belassen auf dem Haar bestimmt ist, nach einem der vorstehenden Ansprüche, wobei die Zusammensetzung ferner Propylenglycol umfasst.

5. Wässrige Zusammensetzung, die zum Belassen auf dem Haar bestimmt ist, nach einem der vorstehenden Ansprüche, wobei die Zusammensetzung ferner 2-Hydroxyethylharnstoff umfasst.

6. Wässrige Zusammensetzung, die zum Belassen auf dem Haar bestimmt ist, nach einem der vorstehenden Ansprüche, umfassend 0,5 % bis 2 % Salicylsäure in Kombination mit 0,2 % bis 10 % 2-Hydroxyethylharnstoff.

7. Wässrige Zusammensetzung, die zum Belassen auf dem Haar bestimmt ist, nach einem der vorstehenden Ansprüche, wobei die Zusammensetzung ferner Materialien umfasst, die ausgewählt sind aus der Gruppe bestehend aus Silikonen, Konditionierungsmaterialien, Lösemitteln, Rheologiemodifikatoren, Verdickungsmitteln, Haargesundheitswirkstoffen, Antischuppenwirkstoffen, Antioxidationsmitteln, Pigmenten, Schleifmitteln, Absorptionsmitteln, biologischen Wirkstoffen, Puffersubstanzen, Chelatbildnern, Trübungsmitteln, pH-Wert-Reglern und Mischungen davon.

8. Wässrige Zusammensetzung, die zum Belassen auf dem Haar bestimmt ist, nach einem der vorstehenden Ansprüche, wobei die Zusammensetzung ferner ein System eines kationenaktiven Tensids umfasst.

9. Wässrige Zusammensetzung, die zum Belassen auf dem Haar bestimmt ist, nach einem der vorstehenden Ansprüche, wobei die Zusammensetzung ferner ein System eines nichtionischen Tensids umfasst.

10. Verwenden einer wässrigen Zusammensetzung, die zum Belassen auf dem Haar bestimmt ist, zum Reduzieren von Haarkraus, wobei die wässrige Zusammensetzung, die zum Belassen auf dem Haar bestimmt ist, von zu 0,15 Gew.-% bis 12,0 Gew.-% der wässrigen Zusammensetzung, die zum Belassen auf dem Haar bestimmt ist, ein Feuchtigkeitsregulierungsmaterial oder eine Mischung von Feuchtigkeitsregulierungsmaterialien umfasst, wobei das Feuchtigkeitsregulierungsmaterial 2-Hexyl-1-decanol in Kombination mit Salicylsäure umfasst, wobei die Zusammensetzung, die zum Belassen auf dem Haar bestimmt ist, ferner eine Gelmatrix umfasst, umfassend:

   i.) von zu 0,1 Gew.-% bis 20 Gew.-% der wässrigen Zusammensetzung, die zum Belassen auf dem Haar bestimmt ist, eine oder mehrere Fettverbindungen mit hohem Schmelzpunkt, wobei die eine oder die mehreren Fettverbindungen mit hohem Schmelzpunkt einen Schmelzpunkt von 25 °C oder höher aufweisen;
   ii.) von zu 0,1 Gew.-% bis 10 Gew.-% der Zusammensetzung, die zum Belassen auf dem Haar bestimmt ist, ein kationenaktives Tensidsystem; und
   iii.) wenigstens zu 20 Gew.-% der Zusammensetzung, die zum Belassen auf dem Haar bestimmt ist, einen wässrigen Träger.

**Revendications**

1. Composition aqueuse sans rinçage pour cheveux pour la réduction de frisotis des cheveux comprenant de 0,15 % à 12,0 % d'un matériau de régulation de l'humidité ou d'un mélange de matériaux de régulation de l'humidité en poids de la composition aqueuse sans rinçage pour cheveux, dans laquelle le matériau de régulation de l'humidité comprend du 2-hexyl-1-décanol en combinaison avec de l'acide salicylique ; dans laquelle la composition sans rinçage comprend en outre une matrice de gel comprenant :

   i.) de 0,1 % à 20 % d'un ou plusieurs composés gras à point de fusion élevé, en poids de ladite composition aqueuse sans rinçage pour cheveux, dans laquelle le ou les composés gras à point de fusion élevé ont un point de fusion de 25 °C ou plus ;
   ii.) de 0,1 % à 10 % d'un système tensioactif cationique, en poids de ladite composition aqueuse sans rinçage pour cheveux ; et

iii.) au moins 20 % d'un véhicule aqueux, en poids de ladite composition aqueuse sans rinçage pour cheveux.

2. Composition aqueuse sans rinçage pour cheveux selon la revendication 1, dans laquelle la concentration en matériau de régulation de l'humidité ou la concentration en mélange de matériaux de régulation de l'humidité va de 0,2 % à 5 %, de préférence de 0,5 % à 4 %, plus préférablement de 1,02 % à 3,0 %, en poids total de la composition aqueuse sans rinçage pour cheveux.

3. Composition aqueuse sans rinçage pour cheveux selon l'une quelconque des revendications précédentes, dans laquelle la composition comprend en outre de l'isostéarate d'isostéaryle.

4. Composition aqueuse sans rinçage pour cheveux selon l'une quelconque des revendications précédentes, dans laquelle la composition comprend en outre du polyproylène glycol.

5. Composition aqueuse sans rinçage pour cheveux selon l'une quelconque des revendications précédentes, dans laquelle la composition comprend en outre de la 2-hydroxyéthylurée.

6. Composition aqueuse sans rinçage pour cheveux selon l'une quelconque des revendications précédentes, comprenant 0,5 % à 2 % d'acide salicylique en combinaison avec 0,2 % à 10 % de 2-hydroxyéthylurée.

7. Composition aqueuse sans rinçage pour cheveux selon l'une quelconque des revendications précédentes, dans laquelle la composition comprend en outre des matériaux choisis dans le groupe constitué de silicones, matériaux revitalisants, solvants, modificateurs de rhéologie, épaississants, actifs pour la santé des cheveux, actifs antipelliculaires, antioxydants, pigments, abrasifs, absorbants, actifs biologiques, agents tampons, agents chélatants, agents opacifiants, ajusteurs de pH et mélanges de ceux-ci.

8. Composition aqueuse sans rinçage pour cheveux selon l'une quelconque des revendications précédentes, dans laquelle la composition comprend en outre un système tensioactif cationique.

9. Composition aqueuse sans rinçage pour cheveux selon l'une quelconque des revendications précédentes, dans laquelle la composition comprend en outre un système tensioactif non ionique.

10. Utilisation d'une composition aqueuse sans rinçage pour cheveux permettant de réduire les frisottis des cheveux, dans laquelle la composition aqueuse sans rinçage pour cheveux comprend de 0,15 % à 12,0 % d'un matériau de régulation de l'humidité ou d'un mélange de matériaux de régulation de l'humidité en poids de la composition aqueuse sans rinçage pour cheveux, dans laquelle le matériau de régulation de l'humidité comprend du 2-hexyl-1-décanol en combinaison avec de l'acide salicylique, dans laquelle la composition sans rinçage comprend en outre une matrice de gel comprenant :

i.) de 0,1 % à 20 % d'un ou plusieurs composés gras à point de fusion élevé, en poids de ladite composition aqueuse sans rinçage pour cheveux, dans laquelle le ou les composés gras à point de fusion élevé ont un point de fusion de 25 °C ou plus ;
ii.) de 0,1 % à 10 % d'un système tensioactif cationique, en poids de ladite composition aqueuse sans rinçage pour cheveux ; et
iii.) au moins 20 % d'un véhicule aqueux, en poids de ladite composition aqueuse sans rinçage pour cheveux.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120093751 A1
- JP 2014097931 A
- JP S63156711 A
- US 20080038206 A1
- US 34584 A
- US 5104646 A
- US 5106609 A

- US 4476282 A
- US 20070276087
- US 20110305778 A1
- US 2694668 A
- US 3152046 A
- US 4089945 A
- US 4885107 A

**Non-patent literature cited in the description**

- *CHEMICAL ABSTRACTS,* 6772-38-3
- Encyclopedia of Polymer Science and Engineering. John Wiley & Sons, Inc, 1989, vol. 15, 204-308
- **SMITH, A. L.** The Analytical Chemistry of Silicones. John Wiley & Sons, Inc, 1991
- **CREPALDI, E L ; PAVA, P C ; TRONTO, J ; VALIM.** *J B J. Colloid Interfac. Sci.,* 2002, vol. 248, 429-42

- **MORIOKA, H. ; TAGAYA, H. ; KARASU, M ; KADOKAWA, J ; CHIBA, K.** *Inorg. Chem,* 1999, vol. 38, 4211-6
- Cosmetic, Toiletry, and Fragrance Association, Inc. 2004